(19) Europäisches Patentamt
European Patent Office
Office européen des brevets

(11) Veröffentlichungsnummer: **0 090 247**
**B1**

(12) **EUROPÄISCHE PATENTSCHRIFT**

(45) Veröffentlichungstag der Patentschrift: **23.07.86**

(21) Anmeldenummer: **83102486.4**

(22) Anmeldetag: **14.03.83**

(51) Int. Cl.[4]: **C 07 D 239/34,** C 07 D 239/36, C 07 D 239/52, C 07 D 239/56, C 07 D 239/70, A 01 N 47/18

(54) **Substituierte N,N-Dimethyl-O-pyrimidin-4-yl-carbamin-säureester, Verfahren zu ihrer Herstellung und ihre Verwendung als Schädlingsbekämpfungsmittel.**

(30) Priorität: **25.03.82 DE 3211035**

(43) Veröffentlichungstag der Anmeldung: **05.10.83 Patentblatt 83/40**

(45) Bekanntmachung des Hinweises auf die Patenterteilung: **23.07.86 Patentblatt 86/30**

(84) Benannte Vertragsstaaten: **AT BE CH DE FR GB IT LI NL**

(56) Entgegenhaltungen:
**EP-A-0 002 200**
**EP-A-0 008 762**
**EP-A-0 022 511**
**FR-A-1 060 289**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Hammann, Ingeborg, Dr., Lutherstrasse 22, D-4330 Mülheim/Ruhr (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

**EP 0 090 247 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue substituierte N,N-Dimethyl-O-pyrimidin-4-yl-carbaminsäureester, mehrere Verfahren zu ihrer Herstellung sowie ihre Verwendung als Schädlingsbekämpfungsmittel, insbesondere als Insektizide.

Es ist bekannt, daß bestimmte N,N-Dialkyl-O-pyrimidinyl-carbaminsäureester, wie z.B. N,N-Dimethyl-O-(2-isopropyl-6-methyl-pyrimidin-4-yl)- und N,N-Dimethyl-O-(2-methylthio-6-methylpyridimin-4-yl)-carbaminsäureester, insektizide Eigenschaften aufweisen (vergleiche FR—A—1 443 910 und US—A—2 694 712). Aus EP—A—2 200, EP—A—8 762 und EP—A—22 511 sind ebenfalls eine Reihe von insektizid wirksamen N,N-Dimethyl-bzw.-Dialkylpyrimidinylcarbaminsäureesterderivaten bekannt. Die insektizide Wirkung dieser Verbindungen ist jedoch, insbesondere bei niedrigen Wirkstoffkonzentrationen und Aufwandmengen nicht immer zufriedenstellend.

Es wurden gefunden:

1) Substituierte N,N-Dimethyl-O-pyrimidin-4-yl-carbaminsäureester der Formel I

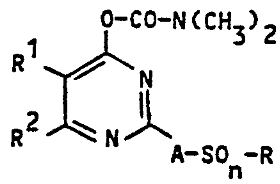

(I)

in welcher

R für Alkyl mit 1 bis 6 Kohlenstoffatomen steht,

$R^1$ für Wasserstoff oder für Alkyl, Alkoxy, Alkylthio, Alkylsulfinyl oder Alkylsulfonyl, jeweils mit 1 bis 6 Kohlenstoffatomen, die gegebenenfalls durch Halogen, $C_{1-2}$-Alkoxy oder $C_{1-2}$-Alkylthio substituiert sein können steht,

$R^2$ für Wasserstoff oder Alkyl mit 1 bis 6 Kohlenstoffatomen das gegebenenfalls durch Halogen insbesondere Chlor oder Fluor, $C_{1-2}$-Alkoxy oder $C_{1-2}$-Alkylthio substituiert sein kann steht, oder

$R^1$ und $R^2$ gemeinsam für Alkylen mit 2 bis 4 Kohlenstoffatomen das gegebenenfalls durch Halogen insbesondere Chlor oder Fluor, $C_{1-2}$-Alkyl, $C_{1-2}$-Alkylthio substituiert sein kann stehen,

A für geradkettiges oder verzweigtes Alkylen mit 2 bis 6 Kohlenstoffatomen steht, und

n für 0, 1 oder 2 steht,

2) Verfahren zur Herstellung der neuen substituierten N,N-Dimethyl-O-pyrimidin-4-yl-carbaminsäureester der Formel (I).

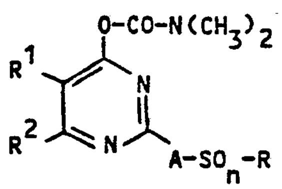

(I)

in welcher

R für Alkyl mit 1 bis 6 Kohlenstoffatomen steht,

$R^1$ für Wasserstoff oder für Alkyl, Alkoxy, Alkylthio, Alkylsulfinyl oder Alkylsulfonyl, jeweils mit 1 bis 6 Kohlenstoffatomen, die gegebenenfalls durch Halogen, $C_{1-2}$-Alkoxy oder $C_{1-2}$-Alkylthio substituiert sein können steht,

$R^2$ für Wasserstoff oder Alkyl mit 1 bis 6 Kohlenstoffatomen das gegebenenfalls durch Halogen insbesondere Chlor oder Fluor, $C_{1-2}$-Alkoxy oder $C_{1-2}$-Alkylthio substituiert sein kann steht, oder

$R^1$ und $R^2$ gemeinsam für Alkylen mit 2 bis 4 Kohlenstoffatomen das gegebenenfalls durch Halogen insbesondere Chlor oder Fluor, $C_{1-2}$-Alkyl, $C_{1-2}$-Alkylthio substituiert sein kann stehen,

A für geradkettiges oder verzweigtes Alkylen mit 2 bis 6 Kohlenstoffatomen steht, und

n für 0, 1 oder 2 steht,

dadurch gekennzeichnet, daß man

a) Hydroxy-pyrimidine der Formel II

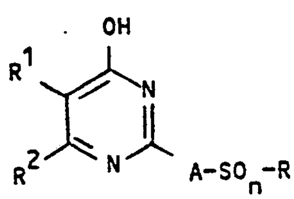

(II)

in welcher

R, $R^1$, $R^2$, A und n die oben angegebene Bedeutungen haben, mit N,N-Dimethyl-carbaminsäurehalogeniden der Formel III

$$Hal—CO—N\ (CH_3)_2 \qquad (III)$$

in welcher

Hal für Chlor oder Brom steht, gegebenenfalls in Gegenwart eines Säureakzeptors und gegebenenfalls unter Verwendung eines Verdünnungsmittels umsetzt; oder

b) Hydroxy-pyrimidine der Formel (II), in welcher

R, $R^1$, $R^2$, A und n die oben angegebenen Bedeutung haben, mit Phosgen und anschließend mit Dimethylamin, gegebenenfalls in Gegenwart eines Säureakzeptors und gegenbenenfalls unter Verwendung eines Verdünnungsmittels umsetzt; oder

c) zum Erhalt von Verbindungen der Formel (I),

in welcher

n für 1 oder 2 steht, und/oder

$R^1$ für Alkylsulfinyl oder -sulfonyl jeweils mit 1 bis 6 Kohlenstoffatomen steht,

Verbindungen der Formel (I), in welcher

n für 0 steht, und/oder

$R^1$ für Alkylthio mit 1 bis 6 Kohlenstoffatomen steht,

mit Oxydationsmitteln gegebenenfalls in Gegenwart eines Verdünnungsmittels umsetzt; oder

d) N,N-Dimethyl-O-pyrimidin-4-yl-carbaminsäureester der Formel IV

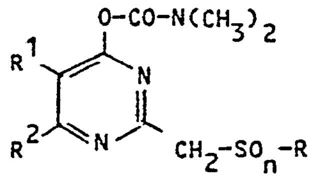

(IV)

in welcher

$R^1$, $R^2$, R und n die oben angegebenen Bedeutungen haben, mit Alkylhalogeniden der Formel V

$$R^3\text{—Hal} \qquad (V)$$

in welcher

$R^3$ für Alkyl steht und

Hal für Chlor, Brom oder lod steht, gegebenenfalls in Gegenwart von Säureakzeptoren und gegebenenfalls in Gegenwart von Verdünnungsmitteln umsetzt.

3) Hydroxypyrimidine der Formel (II)

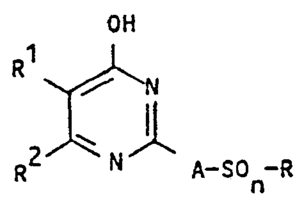

(II)

in welcher

A, R, $R^1$, $R^2$ und n die in Anspruch 1 angegebenen Bedeutungen haben.

4) Verfahren zur Herstellung der neuen Hydroxypyrimidine der Formel II, gemäß Anspruch 3, dadurch gekennzeichnet, daß man Essigsäureester der Formel VI

$$R^1\text{—}CH_2\text{—COO } R^4 \qquad (VI)$$

in welcher

$R^1$ die oben angegebene Bedeutung besitzt und

$R^4$ für Alkyl mit 1—4 C—Atomen steht

mit Estern der Formel VII

$$R^2\text{—COOR}^5 \qquad (VII)$$

in welcher

$R^2$ die oben angegebene Bedeutung besitzt und

$R^5$ für Alkyl mit 1—4 C-Atomen steht,

sowie mit Amidinen der Formel VIII

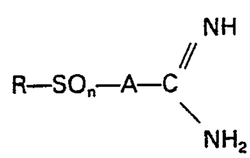

(VII)

in welcher

R, A und n die oben angegebene Bedeutungen haben,

oder deren Hydrohalogeniden, gegebenenfalls in Gegenwart von Säureakzeptron und gegebenenfalls in Gegenwart von Verdünnungsmitteln umsetzt.

Die neuen substituierten N,N-Dimethyl-O-pyrimidin-4-yl-carbaminsäureester der Formel (I) zeichen sich durch hohe Wirksamkeit als Schädlingsbekämpfungsmittel, insbesondere durch hervorragende insektizide Wirkung aus.

Überraschenderweise zeigen die erfindungsgemäßen substituierten N,N-Dimethyl-O-pyrimidin-4-yl-carbaminsäureester eine erheblich höhere insektizide Wirkung als die aus dem Stand der Technik bekannten Verbindungen, wie z.B. N,N-Dimethyl-O-(2-isopropyl-6-methyl-pyrimidin-4-yl)- und N,N-Dimethyl-O-(2-methylthio-6-methyl-pyrimidin-4-yl)-carbaminsäureester, welche Verbindungen analoger Konstitution und gleicher Wirkungsrichtung sind.

Besonders bevorzugt sind die Verbindungen der Formel (I), in welcher

R für Methyl, Ethyl, n-Propyl, i-Propyl, n-Butyl, i-Butyl, sec.-Butyl, und tert.-Butyl steht,

$R^1$ für Wasserstoff, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy, tert.-Butoxy, Methylthio, Ethylthio, n-Propylthio, i-Propylthio, n-Butylthio, i-Butylthio, sec.-Butylthio, tert.-Butylthio, Methyl-, Ethyl-, n-Propyl-, i-Propyl-, n-Butyl-, i-Butyl-, sec.-Butyl- und tert.-Butyl-sulfinyl bzw.-sulfonyl steht,

$R^2$ für Wasserstoff, Methyl, Ethyl, n-Propyl, i-Propyl, n-Butyl, i-Butyl, sec.-Butyl und tert.-Butyl steht oder

$R^1$ und $R^2$ gemeinsam für Alkylen mit 2 bis 4 Kohlenstoffatomen stehen,

A für geradkettiges oder verzweigtes Alkylen mit 2 bis 6 Kohlenstoffatomen steht, und

n für 0, 1 oder 2 steht.

Verwendent man beispielsweise bei Verfahren (a) N,N-Dimethyl-carbaminsäurechlorid, bei Verfahren (b) Phosgen und Dimethylamin, und bei beiden Verfahren 2-(2-Methylsulfonyl-ethyl)-5,6-dimethyl-4-hydroxy-pyrimidin als Ausgangstoffe, so können die entsprechenden Reaktionen durch folgende Formelschemata skizziert werden:

a)

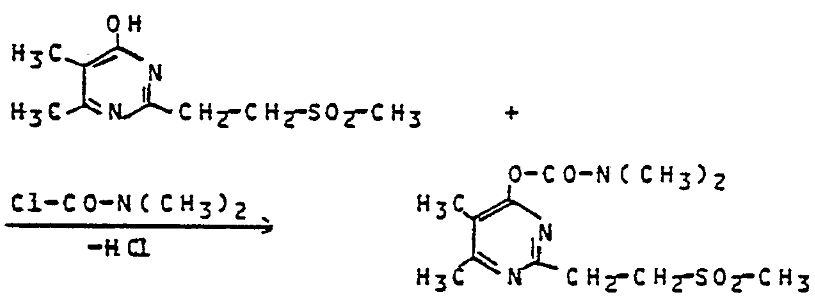


b)

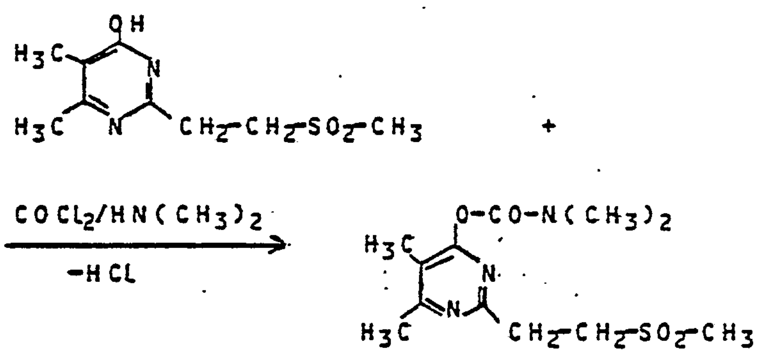


Verwendent man beispielsweise bei Verfahren (c) N,N-Dimethyl-O-(2-(2-methylthio-ethyl)-5,6-dimethyl-pyrimidin-4-yl)-carbaminsäureester und Wasserstoffperoxid als Ausgangstoffe, so kann die entsprechende Reaktion durch folgendes Formelschema skizziert werden:

c)

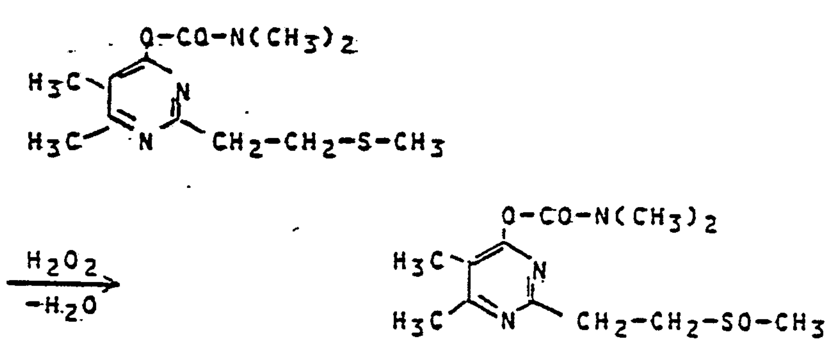

Verwendet man beispielsweise bei Verfahren (d) N,N-Dimethyl-O-(2-(Methylsulfonylmethyl)-5-methoxy-6-methylpyrimidin-4-yl)-carbaminsäureester und Methyliodid als Ausgangsstoffe, so kann die entsprechende Reaktion durch folgendes Formelschema skizziert werden:

d)

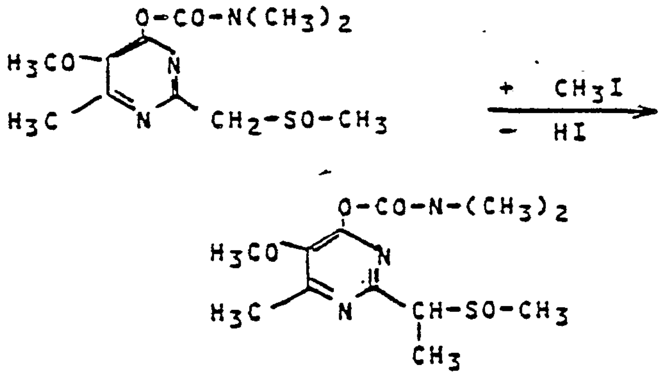


Die bei den Verfahren (a) und (b) als Ausgangsstoffe zu verwendenden Hydroxypyrimidine sind durch die Formel (II) definiert. Vorzugsweise stehen darin R, $R^1$, $R^2$, A und n für diejenigen Reste bzw. Indices, welche bei der Definition der Reste bzw. Indices in Formel (I) als bevorzugt genannt wurden.

Als Beispiele für Verbindungen der Formel (II) seien genannt:

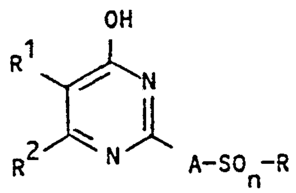

(II)

TABELLE 1

| $R^1$ | $R^2$ | R | A | n |
|---|---|---|---|---|
| $CH_3$ | $CH_3$ | $CH_3$ | $-CH_2-CH_2-$ | 0 |
| $CH_3$ | $CH_3$ | $CH_3$ | $-CH_2-CH_2-$ | 1 |
| $CH_3$ | $CH_3$ | $CH_3$ | $-CH_2-CH_2-$ | 2 |
| $CH_3$ | $CH_3$ | $CH_3$ | $-CH(CH_3)-$ | 2 |
| $-CH_2-CH_2-CH_2-$ | | $CH_3$ | $-CH(CH_3)-$ | 2 |
| $-CH_2-CH_2-CH_2-$ | | $CH_3$ | $-CH(C_2H_5)-$ | 2 |
| $-CH_2-CH_2-CH_2-$ | | $CH_3$ | $-CH_2-CH_2-$ | 0 |
| $-OCH_3$ | H | $CH_3$ | $-CH_2-CH_2-$ | 0 |
| $CH_3$ | $CH_3$ | $CH_3$ | $-CH(CH_3)-$ | 0 |
| $-OCH_3$ | H | $CH_3$ | $-CH(CH_3)-$ | 0 |
| $-CH_2-CH_2-CH_2-$ | | $CH_3$ | $-CH(CH_3)-$ | 0 |
| $CH_3$ | $CH_3$ | $CH_3$ | $-CH(CH_3)-$ | 1 |
| $-OCH_3$ | H | $CH_3$ | $-CH(CH_3)-$ | 1 |
| $-OCH_3$ | H | $CH_3$ | $-CH(CH_3)-$ | 2 |

TABELLE 1 (Fortsetzung)

| $R^1$ | $R^2$ | R | A | n |
|---|---|---|---|---|
| $—CH_2—CH_2—CH_2—$ | | $CH_3$ | $—CH(CH_3)—$ | 1 |
| $—OC_2H_5$ | H | $CH_3$ | $—CH(CH_3)—$ | 0 |
| $—OC_2H_5$ | H | $CH_3$ | $—CH(CH_3)—$ | 1 |
| $—OC_2H_5$ | H | $CH_3$ | $—CH(CH_3)—$ | 2 |
| $—OCH_3$ | H | $CH_3$ | $—CH_2—CH_2—$ | 1 |
| $—OCH_3$ | H | $CH_3$ | $—CH_2—CH_2—$ | 2 |
| $—OC_3H_7$-i | H | $CH_3$ | $—CH_2—CH_2—$ | 0 |
| $—OC_2H_5$ | H | $CH_3$ | $—CH_2—CH_2—$ | 0 |
| $CH_3$ | $CH_3$ | $C_2H_5$ | $—CH_2—CH_2—$ | 0 |
| $—OCH_3$ | H | $C_2H_5$ | $—CH_2—CH_2—$ | 0 |
| $CH_3$ | $CH_3$ | n-$C_3H_7$ | $—CH_2—CH_2—$ | 0 |
| $—OC_3H_7$-n | H | $CH_3$ | $—CH(CH_3)—$ | 0 |
| $—OC_3H_7$-n | H | $CH_3$ | $—CH_2—CH_2—$ | 0 |
| $—OC_3H_7$-n | H | $CH_3$ | $—C(CH_3)_2—CH_2—$ | 0 |
| $—OC_3H_7$-n | H | $CH_3$ | $—CH(CH_3)—CH_2—$ | 0 |
| $—OCH_3$ | H | n-$C_3H_7$ | $—CH_2—CH_2—$ | 0 |
| $CH_3$ | $CH_3$ | tert.-$C_4H_9$ | $—CH_2—CH_2—$ | 0 |
| $—OCH_3$ | H | tert.-$C_4H_9$ | $—CH_2—CH_2—$ | 0 |
| $—OCH_3$ | H | $CH_3$ | $—CH_2—CH_2—CH_2—$ | 0 |
| $—OCH_3$ | H | $CH_3$ | $—CH_2—CH_2—CH_2—$ | 1 |
| $—OCH_3$ | H | $CH_3$ | $—CH_2—CH_2—CH_2—$ | 2 |
| $CH_3$ | H | $CH_3$ | $—CH_2—CH_2—$ | 0 |
| $CH_3$ | H | $CH_3$ | $—CH(CH_3)—$ | 0 |
| $C_2H_5$ | H | $CH_3$ | $—CH_2—CH_2—$ | 0 |
| $C_2H_5$ | H | $CH_3$ | $—CH(CH_3)—$ | 0 |
| i-$C_3H_7$ | H | $CH_3$ | $—CH_2—CH_2—$ | 0 |
| i-$C_3H_7$ | H | $CH_3$ | $—CH(CH_3)—$ | 0 |
| H | $CH_3$ | $CH_3$ | $—CH_2—CH_2—$ | 0 |
| $—OC_2H_5$ | H | $CH_3$ | $—CH_2—CH_2—$ | 1 |
| $—OC_2H_5$ | H | $CH_3$ | $—CH_2—CH_2—$ | 2 |
| $—SCH_3$ | H | $CH_3$ | $—CH_2—CH_2—$ | 0 |

TABELLE 1 (Fortsetzung)

| $R^1$ | $R^2$ | R | A | n |
|---|---|---|---|---|
| $-SCH_3$ | H | $CH_3$ | $-CH(CH_3)-$ | 0 |
| $-OCH_3$ | H | $CH_3$ | $-C(CH_3)_2-CH_2-$ | 0 |
| $CH_3$ | $CH_3$ | $CH_3$ | $-C(CH_3)_2-CH_2-$ | 0 |
| $-OCH_3$ | H | $CH_3$ | $-C(CH_3)_2-$ | 0 |
| $CH_3$ | $CH_3$ | $CH_3$ | $-C(CH_3)_2-$ | 0 |
| $-OCH_3$ | H | $CH_3$ | $-C(CH_3)_2-$ | 1 |
| $-OCH_3$ | H | $CH_3$ | $-C(CH_3)_2-$ | 2 |
| $CH_3$ | $CH_3$ | $CH_3$ | $-C(CH_3)_2-$ | 1 |
| $CH_3$ | $CH_3$ | $CH_3$ | $-C(CH_3)_2-$ | 2 |
| $-OCH_3$ | H | $CH_3$ | $-CH(CH_3)-CH_2-$ | 0 |
| $CH_3$ | $CH_3$ | $CH_3$ | $-CH(CH_3)-CH_2-$ | 0 |
| H | tert.-$C_4H_9$ | $CH_3$ | $-CH_2-CH_2-$ | 0 |

Die Hydroxy-pyrimidine der Formel (II) sind noch nicht in der Literatur beschrieben. Man erhält diese Verbindungen durch Umsetzung von Essigsäureestern, der Formel (VI), beispielsweise von Methoxyessigsäuremethylester, mit Estern, der Formel (VII), wie z.B. Ameisensäuremethylester, in Gegenwart von Basen, wie z.B. Natriummethylat, und gegebenenfalls unter Verwendung von Verdünnungsmitteln, wie z.B. Methanol, bei Temperaturen zwischen 0 und 50°C, und Umsetzung der hierbei erhaltenen Produkte mit Amidinen der Formel (VIII) oder deren Hydrochloriden, wie z.b. Methylthio-propionamidinhydrochlorid, ebenfalls bei Temperaturen zwischen 0 und 50°C. Zur Aufarbeitung wird das Lösungsmittel im Vakuum abgezogen, der Rückstand in Wasser gelöst und ein pH-Wert von ungefähr 6 eingestellt. Man extrahiert mit einem mit Wasser nicht mischbaren Lösungsmittel, wie z.B. Methylenchlorid, und destilliert vom Extrakt das Lösungsmittel ab, wobei das Produkt als fester oder öliger Rückstand erhalten wird. Alkylthio-alkylen-hydroxy-pyrimidine der Formel (II) können mit Oxidationsmitteln, wie z.B. Wasserstoffperoxid oder 3-Chlor-perbenzoesäure, bei Temperaturen zwischen 0 und 50°C, gegebenenfalls unter Verwendung von Verdünnungsmitteln, wie z.B. Essigsäure oder Chloroform, zu den entsprechenden Alkylsulfinyl- bzw. Alkylsulfonyl-alkylen-hydroxy-pyrimidinen oxidiert werden.

Als Beispiel für die bei Verfahren (a) zu verwendenden Carbaminsäurehalogenide der Formel (III) sei N,N-Dimethyl-carbaminsäurechlorid genannt. Diese Verbindung und die bei Verfahren (b) einzusetzenden Reaktionskomponenten Phosgen und Dimethylamin sind bekannt.

Die bei Verfahren (c) als Ausgangsstoffe zu verwendenden Verbindungen sind durch die Formel (I) definiert. In dieser Formel haben R, $R^2$ und A vorzugsweise diejenigen Bedeutungen, welche bei der Definition dieser Reste in Formel (I) als bevorzugt gennant wurden; n steht für 0 und/oder $R^1$ für Alkylthio mit 1 bis 6 Kohlenstoffatomen.

Die bei Verfahren (c) einzusetzenden Oxidationsmittel sind Wasserstoffperoxid, Natriumperjodat, Salpetersäure, Halogene wie Chlor bzw. Hypochlorit, Permanganate wie Kaliumpermanganat, Persäuren wie Perbenzoesäure, Chlorperbenzoesäure.

Für den Fall, daß Verbindungen der Formel I, in der n für 1 steht, erhalten werden sollen, sind bevorzugte Oxidationsmittel $H_2O_2$ bzw. $H_2O_2$/Eisessig, Natriumperjodat, Salpetersäure, Halogene. Das Oxidationsmittel wird im molaren Verhältnis von etwa 1:1 zu den Verbindungen der Formel I, in der n = 0, ist zugesetzt.

Für den Fall, daß Verbindungen der Formel I, in der n für 2 steht erhalten werden sollen, sind bevorzugte Oxidationsmittel $H_2O_2$ mit starken Säuren wie $H_2SO_4$ oder Ameisensäure,, Salpetersäure, Hypochlorit, Permanganate wie Kaliumpermanganat, Persäuren wie Perbenzoesäure oder Chlorperbenzosäure. Das Oxidationsmittel wird im molaren Verhältnis von etwa 2:1 zu den Verbindungen der Formel I, in der n = 0 ist, zugesetzt.

Die bei Verfahren (d) als Ausgangsstoffe zu verwendenden N,N-Dimethyl-O-pyrimidin-4-yl-carbaminsäureester sind durch die Formel (IV) allgemein definiert. In dieser Formel haben R, $R^1$, $R^2$ und n vorzugsweise diejenigen Bedeutungen, welche bei der Definition dieser Reste bzw. Indices in Formel (I) als bevorzugt genannt werden.

Als Beispiele für die Verbindungen der Formel (IV) seien genannt:

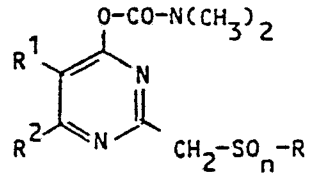

(IV)

TABELLE 2

| $R^1$ | $R^2$ | R | n |
|---|---|---|---|
| $CH_3$ | $CH_3$ | $CH_3$ | 0 |
| $CH_3$ | $CH_3$ | $CH_3$ | 1 |
| $CH_3$ | $CH_3$ | $CH_3$ | 2 |
| $-CH_2-CH_2-CH_2-$ | | $CH_3$ | 0 |
| $-CH_2-CH_2-CH_2-$ | | $CH_3$ | 1 |
| $-CH_2-CH_2-CH_2-$ | | $CH_3$ | 2 |
| $-OCH_3$ | H | $CH_3$ | 0 |
| $-OCH_3$ | H | $CH_3$ | 1 |
| $-OCH_3$ | H | $CH_3$ | 2 |
| $-OC_2H_5$ | H | $CH_3$ | 0 |
| $-OC_2H_5$ | H | $CH_3$ | 1 |
| $-OC_2H_5$ | H | $CH_3$ | 2 |
| $-OC_3H_7$-i | H | $CH_3$ | 0 |
| $CH_3$ | $CH_3$ | $C_2H_5$ | 0 |
| $CH_3$ | $CH_3$ | $C_2H_5$ | 1 |
| $CH_3$ | $CH_3$ | $C_2H_5$ | 2 |
| $-OCH_3$ | H | $C_2H_5$ | 0 |
| $-OCH_3$ | H | $C_2H_5$ | 1 |
| $-OCH_3$ | H | $C_2H_5$ | 2 |
| $-OCH_3$ | H | n-$C_3H_7$ | 0 |
| $-OCH_3$ | H | n-$C_3H_7$ | 1 |
| $-OCH_3$ | H | n-$C_3H_7$ | 2 |
| $-OCH_3$ | H | tert.-$C_4H_9$ | 0 |
| $-OCH_3$ | H | tert.-$C_4H_9$ | 1 |
| $-OCH_3$ | H | tert.-$C_4H_9$ | 2 |
| $CH_3$ | H | $CH_3$ | 0 |

TABELLE 2 (Fortsetzung)

| $R^1$ | $R^2$ | R | n |
|---|---|---|---|
| $CH_3$ | H | $CH_3$ | 1 |
| $CH_3$ | H | $CH_3$ | 2 |
| $C_2H_5$ | H | $CH_3$ | 0 |
| $C_2H_5$ | H | $CH_3$ | 1 |
| $C_2H_5$ | H | $CH_3$ | 2 |
| n-$C_3H_7$ | H | $CH_3$ | 0 |
| n-$C_3H_7$ | H | $CH_3$ | 1 |
| n-$C_3H_7$ | H | $CH_3$ | 2 |
| i-$C_3H_7$ | H | $CH_3$ | 0 |
| i-$C_3H_7$ | H | $CH_3$ | 1 |
| i-$C_3H_7$ | H | $CH_3$ | 2 |
| i-$C_3H_7$ | H | $CH_3$ | 3 |
| H | $CH_3$ | $CH_3$ | 0 |
| H | $CH_3$ | $CH_3$ | 1 |
| H | $CH_3$ | $CH_3$ | 2 |
| —$SCH_3$ | H | $CH_3$ | 0 |
| —$SCH_3$ | H | $CH_3$ | 1 |
| —$SCH_3$ | H | $CH_3$ | 2 |
| H | tert.-$C_4H_9$ | $CH_3$ | 0 |
| H | n-$C_4H_9$ | $CH_3$ | 0 |
| H | i-$C_4H_9$ | $CH_3$ | 0 |
| —$SCH_3$ | $C_2H_5$ | $CH_3$ | 0 |
| $OC_2H_5$ | H | $C_2H_5$ | 0 |

Die Verbindungen der Formel (IV) sind bekannt (vergleiche hierzu DE—A—2 928 185 [Le A 19 743] und DE—A—2 838 359 [Le A 19 079] oder lassen sich auf einfache Weise nach den dort beschriebenen Verfahren herstellen.

Die bei Verfahren (d) weiterhin als Ausgangsstoffe zu verwenden Alkylhalogeniden sind durch die Formel (V) allgemein definiert. Vorzugsweise steht in dieser Formel $R^3$ für $C_1$—$C_5$-Alkyl und Hal für Chlor, Brom oder Iod.

Als Beispiele für die Verbindungen der Formel (IV) seien genannt: Methyl-, Ethyl-, n-Propyl, i-Propyl-, n-Butyl-, i-Butyl-, sec.-Butyl-, tert.-Butyl-, n-Pentyl-, 2-Pentyl-, 3-Pentyl-, 2-Methyl-1-butyl-, 2-Methyl-2-butyl-, 2-Methyl-3-butyl-, 2-Methyl-4-butyl-, 2,2-Dimethyl-1-propyl-chlorid bzw. bromid oder iodid.

Die Verbindungen der Formel (V) sind allgemein bekannte Verbindungen der organischen Chemie.

Die Verfahren (a) bis (d) zur Herstellung den neuen substituierten N,N-Dimethyl-O-pyrimidin-4-yl-carbaminsäureester werden im allgemeinen unter Verwendung von Verdünnungsmittlen durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe,

wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methyl-isopropyl- und Methyl-isobutylketone, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Wird bei Verfahren (c) als Oxidationsmittel Wasserstoffperoxid eingesetzt, kommen vorzugsweise aliphatische Carbonsäuren, wie z.B. Ameisensäure, Essigsäure oder Propionsäure, als Verdünnungsmittel in Frage.

Verfahren (a), (b) und (d) werden im allgemeinen unter Verwendung von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat und Kalium-tert.-butylat, ferner aliphatische, aromatische oder heterocyclische Amine, beispielsweise Triethylamin, Trimethylamin, Dimethyl-anilin, Dimethyl-benzylamin, Pyridin, Diazabicyclooctan, Diazabicyclononan und Diazabicycloundecen.

Die erfindungsgemäßen Verfahren werden im allgemeinen bei Temperaturen zwischen 0 und 150°C durchgeführt. Bevorzugt wird für Verfahren (a) der Temperaturbereich zwischen 20 und 100°C, für Verfahren (b), (c) und (d) der Bereich zwischen 0 und 50°C. Die Umsetzungen werden im allgemeinen bei Normaldruck durchgeführt.

Zur Durchführung von Verfahren (a) bzw. (b) werden auf 1 Mol Hydroxy-pyrimidin der Formel (II) zwischen 1,0 und 1,3 vorzugsweise zwischen 1,0 und 1,15 Mol N,N-Dimethylcarbaminsäurechlorid bzw. Phosgen und Dimethylamin eingesetzt. Die Umsetzung wird im allgemeinen in einem Verdünnungsmittel in Gegenwart eines Säureakzeptors durchgeführt. Nach Ablauf der Reaktion wird filtriert und das Lösungsmittel im Vakuum abdestilliert.

Bei Verfahren (c) werden auf 1 Mol der Verbindung der Formel (I) zwischen 1 und 3 Mol Oxidationsmittel eingesetzt. Die Umsetzung wird im allgemeinen in einem Verdünnungsmittel durchgeführt. Nach Ablauf der Reaktion wird gewaschen, getrocknet, filtriert und das Lösungsmittel im Vakuum abdestilliert.

Zur Durchführung von Verfahren (d) werden auf 1 Mol N,N-Dimethyl-O-pyrimidin-4-yl-carbaminsäureester der Formel (IV) zwischen 1,0 und 1,3 vorzugsweise zwischen 1,0 und 1,15 Mol Alkylhalogenid der Formel (V) eingesetzt. Die Umsetzung wird im allgemeinen in einem Verdünnungsmittel in Gegenwart eines Säureakzeptors durchgeführt.

Die neuen Verbindungen fallen in Form von Ölen an, die sich zum Teil nicht unzersetzt destillieren lassen, jedoch durch sogenanntes 'Andestillieren', d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Verbindungen nach dem Abdestillieren des Lösungsmittel in fester Form anfallen, werden sie durch Umkristallisieren gereinigt. Zur Charakterisierung dient dann der Schmelzpunkt.

Die erfindungsgemäßen substituierten N,N-Dimethyl-O-pyrimidin-4-yl-carbaminsäureester zeichnen sich durch hohe insektizide, insbesondere auch systemische und wurzel-systemische Wirksamkeit aus.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanuara z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularvis, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum

padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp. Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chili spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophiluys spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen

oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwedungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem Gebiet der Tierhaltung und Viehzucht, wobei durch die Bekämpfung der Schädlinge bessere Ergebnisse, z.B. höhere Milchleistungen, höheres Gewicht, längere Lebensdauer usw. erreicht werden können.

Die Anwendung der erfindungsgemäßen Wirkstoffe geschieht auf diesen Gebieten in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

<u>Herstellungsbeispiele</u>

Beispiel 1

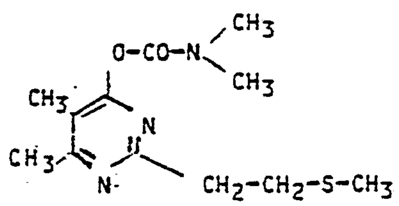

(Verfahren a)

Eine Mischung aus 39,6g (0,2 Mol) 2-(2-Methylthio-ethyl)-4-hydroxy-5,6-dimethylpyrimidin, 41,4 g (0,3 Mol) Kaliumcarbonat, 300 ml Acetonitril und 21,5 g (0,2 Mol) Dimethylcarbamidsäurechlorid wird 48 Stunden bei 60°C gerührt. Dann filtriert man vom Ungelösten, wäscht mit Acetonitril nach und dampft das Filtrat in Vakuum ein. Der Rückstand wird im Hochvakuum bei 60°C andestilliert. Man erhält so 49 g (91% der Theorie) N,N-Dimethyl-O-(2-(2-methylthio-ethyl)-5,6-dimethyl-pyrimidin-4-yl)-carbaminsäureester in Form eines braunen Oeles mit dem Brechungsindex $n_D^{20}$: 1,5350.

Beispiel 2

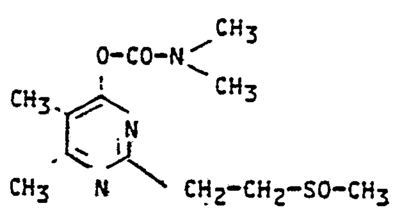

(Verfahren c)

Zu einer Lösung von 13,4 g (0,05 Mol) N,N-Dimethyl-O-(2-(2-methylthio-ethyl)-5,6-dimethylpyrimidin-4-yl)-carbaminsäureester in 100 ml Eisessig tropft man bei 10°C 5,7 g einer 30-%-igen Lösung von Wasserstoffperoxid. Das Gemisch wird 20 Stunden ohne Kühlung nachgerührt und dann mit einer 40-%-igen Natrium-hydrogensulfitlösung versetzt, bis alles Peroxid vernichtet ist. Dann dampft man die Lösung im Vakuum ein, löst den Rückstand in 100 ml Methylenchlorid und schüttelt einmal mit 25 ml 30-%-iger Kaliumcarbonat-lösung aus. Die organische Phase wird über Natriumsulfat getrocknet und im Vakuum eingedampft. Nach Andestillieren erhält man 13,8 g (88% der Theorie). N,N-Dimethyl-O-(2-(2-methylsulfinyl-ethyl)-5,6-dimethyl-pyrimidin-4-yl)-carbaminsäureester als gelbes Oel mit dem Brechungsindex $n_D^{20}$: 1,5372.

Beispiel 3

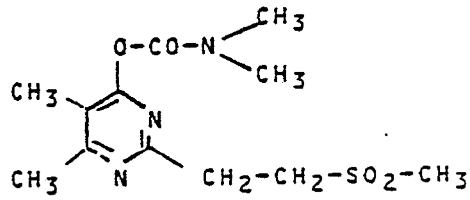

(Verfahren c)

Zu einer Lösung von 13,4 g (0,05 Mol) N,N-Dimethyl-O-(2-(2-methylthio-ethyl)-5,6-dimethyl-pyrimidin-4-yl)-carbaminsäureester in 50 ml Chloroform gibt man unter Kühlen bei 5—10°C eine Lösung von 22,3 g (0,13 Mol) 3-Chlor-peroxibenzoesäure in 200 ml Chloroform. Das Reaktionsgemisch wird 18 Stunden ohne Kühlung nachgerührt und dann filtriert. Dann schüttelt man das Filtrat einmal mit 15 ml einer 30-%-igen Kaliumcarbonatlösung, trocknet die organische Phase über Natriumsulfat und destilliert dann das Lösungsmittel im Vakuum ab. Zurück bleiben 12 g (73% der Theorie) N,N-Dimethyl-O-(2-(2-methylsulfonyl-ethyl)-5,6-dimethyl-pyrimidin-4-yl)-carbaminsäureester in Form farbloser Kristalle mit dem Schmelzpunkt 98°C.

Beispiel 4

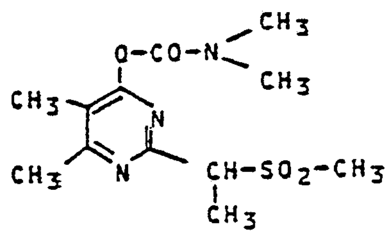

(Verfahren c)

Zu einer Lösung von 20,2 g (0,075 Mol) N,N-Dimethyl-O-(2-(1-methylthio-ethyl)-5,6-dimethyl-pyrimidin-4-yl)-carbaminsäureester in einem Gemisch aus 150 ml Methylenchlorid, 7,5 g Ameisensäure und 0,8 g konz. Schwefelsäure tropft man 24,2 g 30-%-ige Wasserstoffperoxidlösung. Die Reaktionstemperatur steight bis ca. 40°C an. Dann rührt man das Gemisch 18 Stunden ohen Kühlung nach, gibt 40-%-ige Natriumhydrogen-sulfitlösung zu, bis das nicht umgesetzte Peroxid zerstört ist, und schüttelt dann mit 50 ml 30-%-iger Kaliumcarbonatlösung aus. Die organische Phase wird über Natriumsulfat getrocknet und im Vakuum eingedampft. Man erhält so 19 g (84% der Theorie) N,N-Dimethyl-O-(2-(1-methylsulfonyl-ethyl)-5,6-dimethyl-pyrimidin-4-yl)-carbaminsäureester in Form farbloser Kristalle mit dem Schmelzpunkt 128°C.

Beispiel 5.

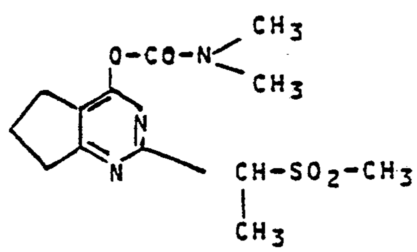

(Verfahren d)

Zu einer Lösung von 5,8 g (0,052 Mol) Kalium-tert.-butylat in 200 ml Tetrahydrofuran gibt man bei 20—25°C 15 g (0,05 Mol) N,N-Dimethyl-O-(2-methylsulfonylmethyl-5,6-trimethylen-pyrimidin-4-yl)-carbaminsäureester und dann 7,8 g (0,055 Mol) Methyliodid und rührt das Gemisch 18 Stunden bei 20—25°C nach. Dann gibt man 500 ml Toluol zu und schüttelt zweimal mit je 100 ml Wasser aus. Die organische Phase wird über Natriumsulfat getrocknet und im Vakuum eingedampft. Zurück bleiben 9,5 g (64% der Theorie) N,N-Dimethyl-O-(2-(1-methylsulfonyl-ethyl)-5,6-trimethylen-pyrimidin-4-yl)-carbaminsäureester in Form beiger Kristalle mit dem Schmelzpunkt 92°C.

Analog einem der Beispiele 1 bis 5 können die folgenden Verbindungen der Formel

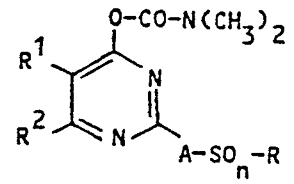

(I)

hergestellt werden:

| Beisp. Nr. | R | $R^1$ | $R^2$ | A | n | Schmelzpunkt [°C]; Brechungsindex |
|---|---|---|---|---|---|---|
| 6 | $CH_3$ | $-CH_2-CH_2-CH_2-$ | | $-CH-$<br>$C_2H_5$ | 2 | 136 |
| 7 | $CH_3$ | $-CH_2-CH_2-CH_2-$ | | $-CH_2-CH_2-$ | 0 | $n_D^{20}$: 1,5490 |
| 8 | $CH_3$ | $CH_3O$ | H | $-CH_2-CH_2-$ | 0 | 44 |
| 9 | $CH_3$ | $CH_3$ | $CH_3$ | $-CH-$<br>$CH_3$ | 0 | 40 |
| 10 | $CH_3$ | $CH_3O$ | H | $-CH-$<br>$CH_3$ | 0 | 112 |
| 11 | $CH_3$ | $-CH_2-CH_2-CH_2-$ | | $-CH-$<br>$CH_3$ | 0 | 39 |
| 12 | $CH_3$ | $CH_3$ | $CH_3$ | $-CH-$<br>$CH_3$ | 1 | $n_D^{22}$: 1,5362 |
| 13 | $CH_3$ | $CH_3O$ | H | $-CH-$<br>$CH_3$ | 1 | 127 |
| 14 | $CH_3$ | $CH_3O$ | H | $-CH-$<br>$CH_3$ | 2 | 144 |
| 15 | $CH_3$ | $-CH_2-CH_2-CH_2-$ | | $-CH-$<br>$CH_3$ | 1 | 71 |
| 16 | $CH_3$ | $C_2H_5O$ | H | $-CH-$<br>$CH_3$ | 0 | $n_D^{21}$: 1,5357 |
| 17 | $CH_3$ | $C_2H_5O$ | H | $-CH-$<br>$CH_3$ | 1 | 68 |
| 18 | $CH_3$ | $C_2H_5O$ | H | $-CH-$<br>$CH_3$ | 2 | 84 |
| 19 | $CH_3$ | $CH_3O$ | H | $-CH_2-CH_2-$ | 1 | |
| 20 | $CH_3$ | $CH_3O$ | H | $-CH_2-CH_2-$ | 2 | |

| Beisp. Nr. | R | $R^1$ | $R^2$ | A | n | Schmelzpunkt [°C]; Brechungsindex |
|---|---|---|---|---|---|---|
| 21 | $CH_3$ | $C_2H_5O$ | H | $-CH_2-CH_2-$ | 0 | |
| 22 | $CH_3$ | $i\text{-}C_3H_7O$ | H | $-CH_2-CH_2-$ | 0 | |
| 23 | $C_2H_5$ | $CH_3$ | $CH_3$ | $-CH_2-CH_2-$ | 0 | |
| 24 | $C_2H_5$ | $CH_3O$ | H | $-CH_2-CH_2-$ | 0 | |
| 25 | $n\text{-}C_3H_7$ | $CH_3$ | $CH_3$ | $-CH_2-CH_2-$ | 0 | |
| 26 | $n\text{-}C_3H_7$ | $CH_3O$ | H | $-CH_2-CH_2-$ | 0 | |
| 27 | $tert.\text{-}C_4H_9$ | $CH_3$ | $CH_3$ | $-CH_2-CH_2-$ | 0 | |
| 28 | $tert.\text{-}C_4H_9$ | $CH_3O$ | H | $-CH_2-CH_2-$ | 0 | |
| 29 | $CH_3$ | $CH_3O$ | H | $-CH_2-CH_2-CH_2-$ | 0 | 28 |
| 30 | $CH_3$ | $CH_3O$ | H | $-CH_2-CH_2-CH_2-$ | 1 | 100 |
| 31 | $CH_3$ | $CH_3O$ | H | $-CH_2-CH_2-CH_2-$ | 2 | 118 |
| 32 | $CH_3$ | $CH_3$ | $CH_3$ | $-CH_2-CH_2-CH_2-$ | 0 | $n_D^{20}$: 1,5317 |
| 33 | $CH_3$ | $CH_3$ | H | $-CH_2-CH_2-$ | 0 | |
| 34 | $CH_3$ | $CH_3$ | H | $-CH-$<br>\|<br>$CH_3$ | 0 | |
| 35 | $CH_3$ | $C_2H_5$ | H | $-CH_2-CH_2-$ | 0 | |
| 36 | $CH_3$ | $C_2H_5$ | H | $-CH-$<br>\|<br>$CH_3$ | 0 | |
| 37 | $CH_3$ | $i\text{-}C_3H_7$ | H | $-CH_2-CH_2-$ | 0 | |
| 38 | $CH_3$ | $i\text{-}C_3H_7$ | H | $-CH-$<br>\|<br>$CH_3$ | 0 | |
| 40 | $CH_3$ | H | $CH_3$ | $-CH_2-CH_2-$ | 0 | |
| 41 | $CH_3$ | $C_2H_5O$ | H | $-CH_2-CH_2-$ | 1 | |
| 42 | $CH_3$ | $C_2H_5O$ | H | $-CH_2-CH_2-$ | 2 | |
| 43 | $CH_3$ | $CH_3S$ | H | $-CH_2-CH_2-$ | 0 | |
| 44 | $CH_3$ | $CH_3S$ | H | $-CH-$<br>\|<br>$CH_3$ | 0 | |
| 45 | $CH_3$ | $CH_3O$ | H | $CH_3$<br>\|<br>$-C-CH_2-$<br>\|<br>$CH_3$ | 0 | |

| Beisp. Nr. | R | $R^1$ | $R^2$ | A | n | Schmelzpunkt [°C]; Brechungsindex |
|---|---|---|---|---|---|---|
| 46 | $CH_3$ | $CH_3$ | $CH_3$ | 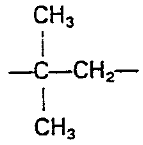 | 0 | |
| 47 | $CH_3$ | $CH_3O$ | H | 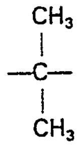 | 0 | |
| 48 | $CH_3$ | $CH_3$ | $CH_3$ | 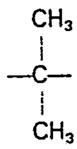 | 0 | |
| 49 | $CH_3$ | $CH_3O$ | H | 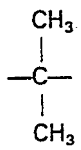 | 1 | |
| 50 | $CH_3$ | $CH_3O$ | H | 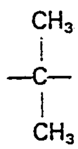 | 2 | |
| 51 | $CH_3$ | $CH_3$ | $CH_3$ | 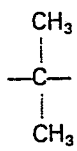 | 1 | |
| 52 | $CH_3$ | $CH_3$ | $CH_3$ | 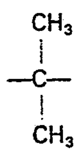 | 2 | |
| 53 | $CH_3$ | $CH_3O$ | H | 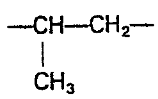 | 0 | |
| 54 | $CH_3$ | $CH_3$ | $CH_3$ | 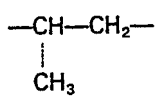 | 0 | |
| 55 | $CH_3$ | H | t.-$C_4H_9$ | $-CH_2-CH_2-$ | 0 | |

Vorprodukte

Die als Ausgangsmaterialien zu verwenden substituierten Hydroxy-pyrimidine der Formel (II) können z.B. wie folgt hergestellt werden:

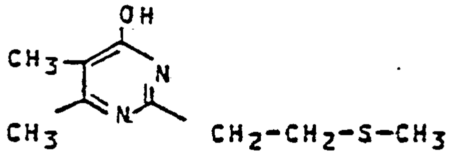

Zu einer Lösung von 54 g (1 Mol) Natriummethanolat in 300 ml Methanol gibt man erst 77,3 g (0,5 Mol) 3-Methylthiopropionamidin-Hydrochlorid und dann 100,7 g (0,5 Mol) 71,4 % ige 2-Methylacetessigsäureethylester. Die Reaktionstemperatur steight dabei bis ca. 35°C an. Dann rührt man das Gemisch 4 Stunden nach und destilliert das Lösungsmittel im Vakuum ab. Der Rückstand wird in 800 ml warmem Wasser gelöst und die Lösung durch Zugabe von konzentrierter Salzsäure auf pH 6 eingestellt. Nach Abkühlen auf 5°C saugt man das ausgefallene Produkt ab. Man erhält so 66 g (67% der Theorie) 2-(Methylthioethyl)-4-hydroxy-5,6-dimethylpyrimidin in Form eines farblosen Pulvers mit dem Schmelzpunkt 120°C.

Analog können die folgenden Verbindungen der Formel:

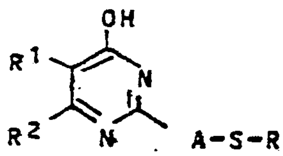

(II a)

hergestellt werden.

| R | $R^1$ | $R^2$ | A | Schmelzpunkt [°C] |
|---|---|---|---|---|
| $CH_3$ | $—CH_2—CH_2—CH_2—$ | | $—CH_2—CH_2—$ | 164 |
| $CH_3$ | $CH_3O$ | H | $—CH_2—CH_2—$ | 155 |
| $CH_3$ | $CH_3$ | $CH_3$ | $—CH(CH_3)—$ | 89 |
| $CH_3$ | $CH_3O$ | H | $—CH(CH_3)—$ | 149 |
| $CH_3$ | $—CH_2—CH_2—CH_2—$ | | $—CH(CH_3)—$ | 154 |
| $CH_3$ | $C_2H_5O$ | H | $—CH(CH_3)—$ | 129 |
| $CH_3$ | $C_2H_5O$ | H | $—CH_2—CH_2—$ | |
| $CH_3$ | $i-C_3H_7O$ | H | $—CH_2—CH_2—$ | |
| $C_2H_5$ | $CH_3$ | $CH_3$ | $—CH_2—CH_2—$ | |
| $C_2H_5$ | $CH_3O$ | H | $—CH_2—CH_2—$ | |
| $n-C_3H_7$ | $CH_3$ | $CH_3$ | $—CH_2—CH_2—$ | |
| $n-C_3H_7$ | $CH_3O$ | H | $—CH_2—CH_2—$ | |
| tert.-$C_4H_9$ | $CH_3$ | $CH_3$ | $—CH_2—CH_2—$ | |
| tert.-$C_4H_9$ | $CH_3O$ | H | $—CH_2—CH_2—$ | |

| R | $R^1$ | $R^2$ | A | Schmelzpunkt [°C] |
|---|---|---|---|---|
| $CH_3$ | $CH_3O$ | H | $-CH_2-CH_2-CH_2-$ | 137 |
| $CH_3$ | $CH_3$ | $CH_3$ | $-CH_2-CH_2-CH_2-$ | 102 |
| $CH_3$ | $CH_3$ | H | $-CH_2-CH_2-$ | |
| $CH_3$ | $CH_3$ | H | $-CH-$<br>\|<br>$CH_3$ | |
| $CH_3$ | $C_2H_5$ | H | $-CH_2-CH_2-$ | |
| $CH_3$ | $C_2H_5$ | H | $-CH-$<br>\|<br>$CH_3$ | |
| $CH_3$ | i-$C_3H_7$ | H | $-CH_2-CH_2-$ | |
| $CH_3$ | i-$C_3H_7$ | H | $-CH-$<br>\|<br>$CH_3$ | |
| $CH_3$ | H | $CH_3$ | $-CH_2-CH_2-$ | |
| $CH_3$ | $CH_3S$ | H | $-CH_2-CH_2-$ | |
| $CH_3$ | $CH_3S$ | H | $-CH-$<br>\|<br>$CH_3$ | |
| $CH_3$ | $CH_3O$ | H | $CH_3$<br>\|<br>$-C-CH_2-$<br>\|<br>$CH_3$ | |
| $CH_3$ | $CH_3$ | $CH_3$ | $CH_3$<br>\|<br>$-C-CH_2-$<br>\|<br>$CH_3$ | |
| $CH_3$ | $CH_3O$ | H | $CH_3$<br>\|<br>$-C-$<br>\|<br>$CH_3$ | |
| $CH_3$ | $CH_3$ | $CH_3$ | $CH_3$<br>\|<br>$-C-$<br>\|<br>$CH_3$ | |
| $CH_3$ | $CH_3O$ | H | $-CH-CH_2-$<br>\|<br>$CH_3$ | |
| $CH_3$ | $CH_3$ | $CH_3$ | $-CH-CH_2-$<br>\|<br>$CH_3$ | |
| $CH_3$ | H | t.-$C_4H_9$ | $-CH_2-CH_2-$ | |

Verwendungsbeispiele

In den nachfolgenden Beispielen werden folgende Verbindungen als Vergleichssubstanzen eingesetzt:

A) 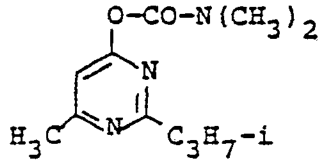

N,N-Dimethyl-O-(2-iso-propyl-6-methyl-pyrimidin-4-yl)-carbaminsäureester
(FR—PS 1 443 910)

B) 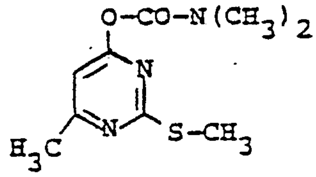

N,N-Dimethyl-O-(2-methylthio-6-methyl-pyrimidin-4-yl)-carbaminsäureester
(US—PS 2 694 712)

Beispiel A

Doralis-Test (systemische Wirkung)

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z.B. die folgenden Verbindungen der Herstellungsbeispiele überlegene Wirkung gegenüber dem Stand der Technik: (10), (9), (11), (13), (12), (17), (15), (14), (5), (4), (6), (8), (1), (7), (2) und (3).

Beispiel B

Grenzkonzentrations-Test/Wurzelsystemische Wirkung

Testinsket: Myzus persicae

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wuzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffes abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: (5), (6), (1), (7), (2), (3), (8), (9), (10), (11), (12), (4), (13), (14), (15), (16), (17) und (18).

Beispiel C

Test mit Lucilia cuprina res.-Larven

Emulgator: 35 Gewichtsteile Ethylenglykolmonomethylether

35 Gewichtsteile Nonylphenolpolyglykolether

Zur Herstellung einer zweckmäßigen Wirkstoffzubereitung vermischt man drei Gewichtssteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigte z.B. die Verbindung des Beispiels (5) bei einer Wirkstoffkonzentration von 1000 ppm eine 100%-ige Abtötung.

**Patentansprüches**

1. Substituierte N,N-Dimethyl-O-pyrimidin-4-yl-carbaminsäureester der Formel I

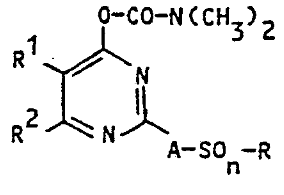

(I)

in welcher

R für Alkyl mit 1 bis 6 Kohlenstoffatomen steht,

$R^1$ für Wasserstoff oder für Alkyl, Alkoxy, Alkylthio, Alkylsulfinyl oder Alkylsulfonyl, jeweils mit 1 bis 6 Kohlenstoffatomen, die gegebenenfalls durch Halogen, $C_{1-2}$-Alkoxy oder $C_{1-2}$-Alkylthio substituiert sein können steht,

$R^2$ für Wasserstoff oder Alkyl mit 1 bis 6 Kohlenstoffatomen das gegebenenfalls durch Halogen insbesondere Chlor oder Fluor, $C_{1-2}$-Alkoxy oder $C_{1-2}$-Alkylthio substituiert sein kann steht, oder

$R^1$ und $R^2$ gemeinsam für Alkylen mit 2 bis 4 Kohlenstoffatomen das gegebenenfalls durch Halogen insbesondere Chlor oder Fluor, $C_{1-2}$-Alkyl, $C_{1-2}$-Alkylthio substituiert sein kann stehen,

A für geradkettiges oder verzweigtes Alkylen mit 2 bis 6 Kohlenstoffatomen steht, und

n für 0, 1 oder 2 steht.

2. Verfahren zur Herstellung der neuen substituierten N,N-Dimethyl-O-pyrimidin-4-yl-carbaminsäureester der Formel (I) nach Anspruch 1

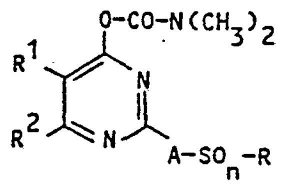

(I)

in welcher

R für Alkyl mit 1 bis 6 Kohlenstoffatomen steht,

$R^1$ für Wasserstoff oder für Alkyl, Alkoxy, Alkylthio, Alkylsulfinyl oder Alkylsulfonyl, jeweils mit 1 bis 6 Kohlenstoffatomen, die gegebenenfalls durch Halogen, $C_{1-2}$-Alkoxy oder $C_{1-2}$-Alkylthio substituiert sein können steht,

$R^2$ für Wasserstoff oder Alkyl mit 1 bis 6 Kohlenstoffatomen das gegebenenfalls durch Halogen insbesondere Chlor oder Fluor, $C_{1-2}$-Alkoxy oder $C_{1-2}$-Alkylthio substituiert sein kann steht, oder

$R^1$ und $R^2$ gemeinsam für Alkylen mit 2 bis 4 Kohlenstoffatomen das gegebenenfalls durch Halogen insbesondere Chlor oder Fluor, $C_{1-2}$-Alkyl, $C_{1-2}$-Alkylthio substituiert sein kann stehen,

A für geradkettiges oder verzweigtes Alkylen mit 2 bis 6 Kohlenstoffatomen steht, und

n für 0, 1 oder 2 steht,

dadurch gekennzeichnet, daß man

a) Hydroxy-pyrimidine der Formel II

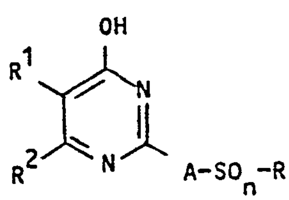

(II)

in welcher

R, $R^1$, $R^2$, A und n die oben angegebene Bedeutungen haben, mit N,N-Dimethyl-carbaminsäurehalogeniden der Formel III

$$Hal—CO—N\ (CH_3)_2 \qquad (III)$$

in welcher

Hal für Chlor oder Brom steht, gegebenenfalls in Gegenwart eines Säureakzeptors und gegebenenfalls unter Verwendung eines Verdünnungsmittels umsetzt; oder

b) Hydroxy-pyrimidine der Formel (II), in welcher

R, $R^1$, $R^2$, A und n die oben angegebenen Bedeutung haben, mit Phosgen und anschließend mit Dimethylamin, gegebenenfalls in Gegenwart eines Säureakzeptors und gegenbenenfalls unter Verwendung eines Verdünnungsmittels umsetzt; oder

c) zum Erhalt von Verbindungen der Formel (I), in welcher

n für 1 oder 2 steht, und/oder

$R^1$ für Alkylsulfinyl oder -sulfonyl jeweils mit 1 bis 6 Kohlenstoffatomen steht,

Verbindungen der Formel (I), in welcher

n für 0 steht, und/oder

$R^1$ für Alkylthio mit 1 bis 6 Kohlenstoffatomen steht,

mit Oxydationsmitteln gegebenenfalls in Gegenwart eines Verdünnungsmittels umsetzt; oder

d) N,N-Dimethyl-O-pyrimidin-4-yl-carbaminsäureester der Formel IV

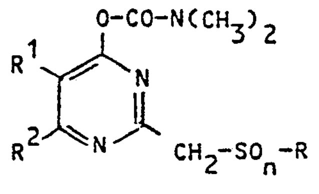

(IV)

in welcher

$R^1$, $R^2$, R und n die oben angegebenen Bedeutungen haben, mit Alkylhalogeniden der Formel V

$$R^3\text{—Hal} \qquad (V)$$

in welcher

$R^3$ für Alkyl steht und

Hal für Chlor, Brom oder Iod steht, gegebenenfalls in Gegenwart von Säureakzeptoren und gegebenenfalls in Gegenwart von Verdünnungsmitteln umsetzt.

3. Hydroxypyrimidine der Formel (II)

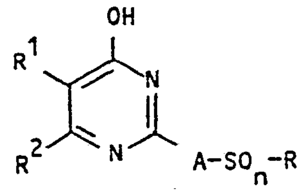

(II)

in welcher

A, R, $R^1$, $R^2$ und n die in Anspruch 1 angegebenen Bedeutungen haben.

4. Verfahren zur Herstellung der neuen Hydroxypyrimidine der Formel II, gemäß Anspruch 3, dadurch gekennzeichnet, daß man Essigsäureester der Formel VI

$$R^1\text{—}CH_2\text{—COO } R^4 \qquad (VI)$$

in welcher

$R^1$ die oben angegebene Bedeutung besitzt und

$R^4$ für Alkyl mit 1—4 C—Atomen steht

mit Estern der Formel VII

$$R^2\text{—COOR}^5 \qquad (VII)$$

in welcher

$R^2$ die oben angegebene Bedeutung besitzt und

$R^5$ für Alkyl mit 1—4 C-Atomen steht,

sowie mit Amidinen der Formel VIII

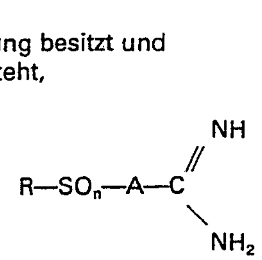

(VIII)

in welcher

R, A und n die oben angegebene Bedeutungen haben,

oder deren Hydrohalogeniden, gegebenenfalls in Gegenwart von Säureakzeptron und gegebenenfalls in Gegenwart von Verdünnungsmitteln umsetzt.

5. Schädlingsbekämpfungsmittel, gekennzeichnet durch einen Gehalt an mindestene einem sustituierten N,N-Dimethyl-O-pyrimidin-4-yl-carbaminsäureester der Formel (I) nach Anspruch 1.

6. Verwendung von substituierten N,N-Dimethyl-O-pyrimidin-4-yl-carbaminsäureester der Formel (I) nach Anspruch 1 zur Bekämpfung von Schädlingen unter Ausschluß von therapeutischen Verwendungen.

7. Verfahren zur Bekämpfung von Schädlingen, dadurch gekennzeichnet, daß man substituierte N,N-Dimethyl-O-Pyrimidin-4-yl-carbaminsäureester der Formel (I) nach Anspruch 1 auf Schädlinge und/oder ihren Lebensraum einwirken läßt unter Ausschluß von therapeutischen Verfahren.

8. Verfahren zur Herstellung von Schädlingsbekämpfungsmitteln, dadurch gekennzeichnet, daß man substituierte N,N-Dimethyl-O-pyrimidin-4-yl-carbaminsäureester der Formel (I) nach Anspruch 1 mit Streckmitteln und/oder oberflächenaktiven Mitteln vermischt.

9. Verwendung von Verbindungen der Formel (I) gemäß Anspruch 1 für Herstellung von mitteln für Bekämpfung von Ekto-und Endoparasiten auf dem bebiet der inerhaltung und Viehtacht.

**Revendications**

1. Esters N,N-diméthyl-O-pyrimidine-4-yl-carbamiques substitués de formule I

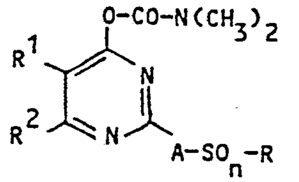

(I)

dans laquelle

R réprésente un groupe alkyle en $C_1$—$C_6$,

$R^1$ représente l'hydrogène ou un groupe alkyle, alcoxy, alkylthio, alkylsulfinyle ou alkylsulfonyle contenant chacun 1 à 6 atomes de carbone et qui peuvent être substitué le cas échéant par des halogènes, des groupes alcoxy en $C_1$—$C_2$ ou alkylthio en $C_1$—$C_2$,

$R^2$ représente l'hydrogène ou un groupe alkyle en $C_1$—$C_6$ qui peut le cas échéant être substitué par des halogènes, en particulier le chlore ou le fluor, des groupes alcoxy en $C_1$—$C_2$ ou alkylthio en $C_1$—$C_2$, ou bien

$R^1$ et $R^2$ forment ensemble un groupe alkylène en $C_2$—$C_4$ qui peut le cas échéant être substitué par des halogènes, en particulier le chlore ou le fluor, des groupes alkyle en $C_1$—$C_2$, alkylthio en $C_1$—$C_2$,

A représente un groupe alkylène à chaîne droite ou ramifiée en $C_2$—$C_6$, et

n est égal à 0, 1 ou 2.

2. Procédé de préparation des nouveaux esters, N,N-diméthyl-O-pyrimidine-4-yl-carbamiques substitués de formule I selon la revendication 1,

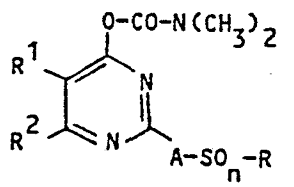

(I)

dans laquelle

R représente un group alkyle en $C_1$—$C_6$,

$R^1$ représente l'hydrogène ou un groupe alkyle, alcoxy, alkylthio, alkylsulfinyle ou alkylsulfonyle contenant chacun 1 à 6 atomes de carbone et qui peuvent cas échéant être substitués par des halogènes, des groupes alcoxy en $C_1$—$C_2$ ou alkylthio en $C_1$—$C_2$,

$R^2$ représente l'hydrogène ou un groupe alkyle en $C_1$—$C_6$ qui peut le cas échéant être substitué par des halogènes, en particulier le chlore ou le fluor, des groupes alcoxy en $C_1$—$C_2$ ou alkylthio en $C_1$—$C_2$, ou bien

$R^1$ et $R^2$ forment ensemble un groupe alkylène en $C_2$—$C_4$ qui peut le cas échéant être substitué par des halogènes, en particulier le chlore ou le fluor, des groupes alkyle en $C_1$—$C_2$, alkylthio en $C_1$—$C_2$,

A représente un groupe alkylène à chaîne droite ou ramifiée en $C_2$—$C_6$, et

n est égal à 0, 1 ou 2,

caractérisé en ce que

a) on fait réagir des hydroxypyrimidines de formule II

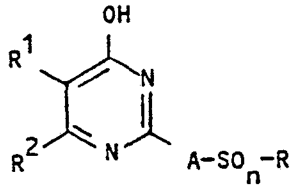

(II)

dans laquelle

R, $R^1$, $R^2$, A et n ont les significations indiquées ci-dessus, avec des halogénures de l'acide N,N-diméthylcarbamique de formule III

$$Hal—CO—N(CH_3)_2 \quad (III)$$

dans laquelle

Hal représente le chlore ou le brome, éventuellement en présence d'un accepteur d'acide et le cas échéant avec utilisation d'un diluant; ou bien

b) on fait réagir des hydroxyprimidines de formule II dans laquelle

R, $R^1$, $R^2$, A et n ont les significations indiquées ci-dessus, avec le phosgène puis la diméthylamine, éventuellement en présence d'un accepteur d'acide et le cas échéant avec utilisation d'un diluant; ou bien

c) pour obtenir des composés de formule I dans laquelle

n est égal à 1 ou 2, et/ou

$R^1$ représente un groupe alkylsulfinyle ou alkylsulfonyle contenant chacun 1 à 6 atomes de carbone, on fait réagir des composés de formule I dans laquelle

n est égal à 0, et/ou

$R^1$ représente un groupe alkylthio en $C_1$—$C_6$,

avec des agents oxydants, éventuellement en présence d'un diluant; ou bien

d) on fait réagir des esters N,N-diméthyl-O-pyrimidine-4-yl-carbamiques de formule IV

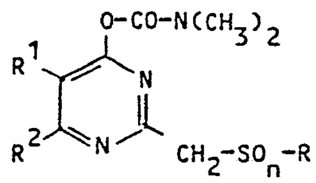

(IV)

dans laquelle

$R^1$, $R^2$, R et n ont les significations indiquées ci-dessus, avec des halogénures d'alkyle de formule V

$$R^3\text{—Hal} \qquad (V)$$

dans laquelle

$R^3$ représente un groupe alkyle, et

Hal représente le chlore, le brome ou l'iode, éventuellement en présence d'accepteurs d'acides et les cas échéant en présence de diluants.

3. Hydroxypyrimidines de formule II

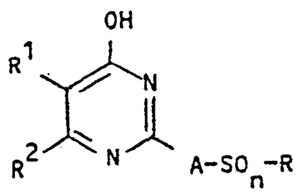

(II)

dans laquelle

A, R, $R^1$, $R^2$ et n ont les significations indiquées dans la revendication 1.

4. Procédé de préparation des nouvelles hydroxypyrimidines de formule II selon la revendication 3, caractérisé en ce que l'on fait réagir des esters acétiques de formule VI

$$R^1\text{—}CH_2\text{—}COOR^4 \qquad (VI)$$

dans laquelle

$R^1$ a les significations indiquées ci-dessus, et

$R^4$ représente un groupe alkyle en $C_1$—$C_4$, avec des esters de formule VII

$$R^2\text{—}COOR^5 \qquad (VII)$$

dans laquelle

$R^2$ a les significations indiquées ci-dessus, et

$R^5$ représente un groupe alkyle en $C_1$—$C_4$, et avec des amidines de formule VIII

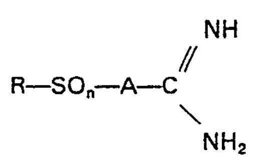

(VIII)

dans laquelle

R, A et n ont les significations indiquées ci-dessus,

ou leurs halogénhydrates, éventuellement en présence d'accepteurs d'acides et le cas échéant en présence de diluants.

5. Produits pesticides, caractérisés en ce qu'ils contiennent au moins un ester N,N-diméthyl-O-pyrimidine-4-yl-carbamiques substitué de formule I selon la revendication 1.

6. Utilisation des esters N,N-diméthyl-O-pyrimidine-4-yl-carbamiques substitués de formule I selon la revendication 1 dans la lutte contre les parasites, à l'exclusion des utilisations thérapeutiques.

7. Procédé pour combattre les parasites, caractérisé en ce que l'on fait réagir sur les parasites et/ou leur habitat des esters N,N-diméthyl-O-pyrimidine-4-yl-carbamiques substitués de formule I selon la revendication 1, à l'exclusion des procédés thérapeutiques.

8. Procédé de préparation de produits pesticides, caractérisé en ce que l'on mélange des esters N,N-diméthyl-O-pyrimidine-4-yl-carbamiques substitués de formule I selon la revendication 1 avec des diluants et/ou des agents tensioactifs.

9. Utilisation des composés de formule I selon la revendication 1 pour la préparation de produits servant à combattre les ecto- et endo-parasites dans le domaine de l'entretien des animaux et de l'élevage du bétail.

**Claims**

1. Substituted N,N-dimethyl-O-pyrimidin-4-yl-carbamic acid esters of the formula I

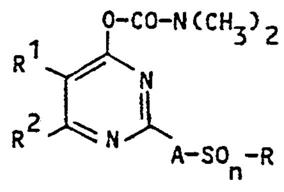

(I)

in which

R represents alkyl with 1 to 6 carbon atoms,

$R^1$ represents hydrogen or alkyl, alkoxy, alkylthio, alkylsulphinyl or alkylsulphonyl, each with 1 to 6 carbon atoms, which can optionally be substituted by halogen, $C_{1-2}$-alkoxy or $C_{1-2}$-alkylthio,

$R^2$ represents hydrogen or alkyl with 1 to 6 carbon atoms which can optionally be substituted by halogen in particular chlorine or fluorine, $C_{1-2}$-alkoxy or $C_{1-2}$-alkylthio, or $R^1$ and $R^2$ together represent alkylene with 2 to 4 carbon atoms which can optionally be substituted by halogen in particular chlorine or fluorine, $C_{1-2}$-alkyl or $C_{1-2}$-alkylthio,

A represents straight-chain or branched alkylene with 2 to 6 carbon atoms, and

n represents 0, 1 or 2.

2. Process for the preparation of the new substituted N,N-dimethyl-O-pyrimidin-4-yl-carbamic acid esters of the formula (I) according to Claim 1

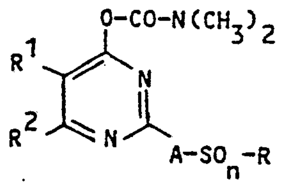

(I)

in which

R represents alkyl with 1 to 6 carbon atoms,

$R^1$ represents hydrogen or alkyl, alkoxy, alkylthio, alkylsulphinyl or alkylsulphonyl, each with 1 to 6 carbon atoms, which can optionally be substituted by halogen, $C_{1-2}$-alkoxy or $C_{1-2}$-alkylthio,

$R^2$ represents hydrogen or alkyl with 1 to 6 carbon atoms which can optionally be substituted by halogen in particular chlorine or fluorine, $C_{1-2}$-alkoxy or $C_{1-2}$-alkylthio, or $R^1$ and $R^2$ together represent alkylene with 2 to 4 carbon atoms which can optionally be substituted by halogen in particular chlorine or fluorine, $C_{1-2}$-alkyl or $C_{1-2}$-alkylthio,

A represents straight-chain or branched alkylene with 2 to 6 carbon atoms, and

n represents 0, 1 or 2,

characterised in that

a) hydroxy-pyrimidines of the formula II

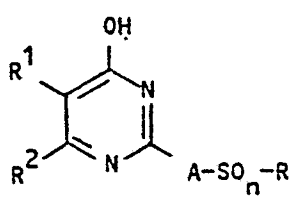

(II)

in which

R, $R^1$, $R^2$, A and n have the aforementioned meanings, are reacted with N,N-dimethyl-carbamic acid halides of the formula III

$$Hal—CO—N(CH_3)_2 \qquad (III)$$

in which

Hal represents chlorine or bromine, if appropriate in the presence of an acid acceptor and if appropriate with the use of a diluent; or

b) hydroxy-pyrmidines of the formula (II), in which

R, $R^1$, $R^2$, A and n have the abovementioned meanings, are reacted with phosgene and then with dimethylamine, if appropriate in the presence of an acid acceptor and if appropriate with the use of a diluent; or

c) to obtain compounds of the formula (I) in which

n represents 1 or 2, and/or

$R^1$ represents alkylsulphinyl or alkylsulphonyl, each with 1 to 6 carbon atoms,

compounds of the formula (I) in which

n represents 0, and/or

$R^1$ represents alkylthio with 1 to 6 carbon atoms, are reacted with oxidation agents, if appropriate in the presence of a diluent; or

d) N,N-dimethyl-O-pyrmidin-4-yl-carbamic acid esters of the formula IV

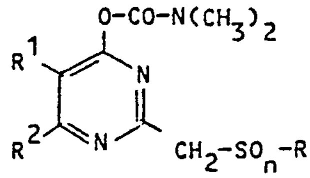

(IV)

in which

$R^1$, $R^2$, R and n have the abovementioned meanings, are reacted with alkyl halides of the formula V

$$R^3\text{—Hal} \quad (V)$$

in which

$R^3$ represents alkyl and

Hal represents chlorine, bromine or iodine, if appropriate in the presence of acid acceptors and if appropriate in the presence of diluents.

3. Hydroxypyrimidines of the formula II

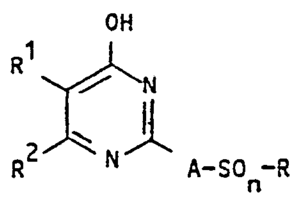

(II)

in which

A, R, $R^1$, $R^2$ and n have the meanings given in Claim 1.

4. Process for the preparation of the new hydroxypyrimidines of the formula II, according to Claim 3, characterised in that acetic acid esters of the formula VI

$$R^1\text{—}CH_2\text{—}COO\ R^4 \quad (VI)$$

in which

$R^1$ has the abovementioned meaning and

$R^4$ represents alkyl with 1—4 C atoms, are reacted with esters of the formula VIII

$$R^2\text{—}COOR^5 \quad (VII)$$

in which

$R^2$ has the abovementioned meaning and

$R^5$ represents alkyl with 1—4 C atoms, and with amidines of the formula VIII

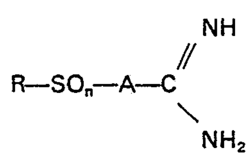

(VIII)

in which

R, A and n have the abovementioned meanings, or hydrohalides thereof, if appropriate in the presence of acid acceptors and if appropriate in the presence of diluents.

5. Pest-combating agents, characterised in that they contain at least one substituted N,N-dimethyl-O-pyrimidin-4-yl-carbamic acid ester of the formula (I) according to Claim 1.

6. Use of substituted N,N-dimethyl-O-pyrimidin-4-yl-carbamic acid esters of the formula (I) according to Claim 1 for combating pests, with the exclusion of therapeutic uses.

7. Method of combating pests, characterised in that substituted N,N-dimethyl-O-pyrimidin-4-yl-

carbamic acid esters of the formula (I) according to Claim are allowed to act on pests and/or their habitat, with the exclusion of therapeutic methods.

8. Process for the preparation of pest-combating agents, characterised in that substituted N,N-dimethyl-O-pyrimidin-4-yl-carbamic acid esters of the formula (I) according to Claim 1 are mixed with extenders and/or surface-active agents.

9. Use of compounds of the formula (I) according to Claim 1 for the preparation of agents for combating ecto- and endo-parasites in the field of animal husbandry and livestock breeding.